# EUROPEAN PATENT APPLICATION

(11) **EP 4 238 520 A1**
(43) Date of publication of application: **06.09.2023**
(21) Application number: 21885362.0
(22) Date of filing: 01.11.2021
(51) Int. Cl.: A61B 18/12

(54) **RADIOFREQUENCY ABLATION INSTRUMENT, CONTROL METHOD THEREFOR AND CONTROL APPARATUS THEREOF, SYSTEM, ELECTRONIC DEVICE AND STORAGE MEDIUM**

(30) Priority: 31.10.2020 CN 202011200579
(71) Applicant: Hangzhou Noya Medtech Co., Ltd., Hangzhou, Zhejiang 310000 (CN)
(72) Inventor: SHI, Shijiang, Hangzhou, Zhejiang 310000 (CN); WANG, Hongfu, Hangzhou, Zhejiang 310000 (CN); GAO, Guoqing, Hangzhou, Zhejiang 310000 (CN); WANG, Yongsheng, Hangzhou, Zhejiang 310000 (CN)
(74) Representative: MERH-IP Matias Erny Reichl Hoffmann Patentanwälte PartG mbB
(86) International application number: PCT/CN2021/127917
(87) International publication number: WO 2022/089636

(57) **Abstract**

A radio-frequency ablation instrument (100) and a control method and a control apparatus (30) thereof, a system, an electronic device (90) and a storage medium are provided. The control method includes: acquiring a current ablation parameter (S701); calculating a power value needed to be outputted in a next cycle according to the current ablation parameter (S702); acquiring an input control voltage corresponding to the power value according to the power value needed to be outputted (S703); and controlling the radio-frequency energy output according to the input control voltage (S704).

## Description

### TECHNICAL FIELD

The present disclosure relates to a radio-frequency ablation instrument and a control method and a control apparatus thereof, a system, an electronic device and a storage medium.

### DESCRIPTION OF THE PRIOR ART

Radio-frequency ablation technology is widely used in the medical field, and radio-frequency ablation instrument is a device employing such a technology. The radio-frequency ablation instrument is configured to generate and adjust radio-frequency energy and ablate tissue with the radio-frequency energy, to achieve the purpose of treatment for a patient. A specific working process includes: positioning an ablation electrode of a radio-frequency ablation instrument in a patient at a site to be ablated, and bringing a neutral electrode into contact with the skin surface of the patient, such that a radio-frequency current can flow through the ablation electrode and the patient's tissue, and a circuit loop is formed between the ablation electrode and the neutral electrode. After the circuit loop is formed, due to the small area of the ablation electrode and the large electrical field strength, significant thermal effect will be exerted on the tissue surrounding the ablation electrode, causing the dehydration, coagulation and necrosis of the tissue, resulting in an ablation point. However, no obvious heating effect will be applied on the patient's skin because of the large area of the neutral electrode.

With the continuous development of the radio-frequency ablation technology, higher requirements are raised for the accuracy and stability of the output of the radio-frequency ablation instrument, to achieve accurate ablation of the target object. The output energy of the ablation instrument is required to reach a certain accuracy, and the stability when continuous output is disturbed also needs to reach a certain degree, to ensure the ablation effect.

In view of the above problems of the radio-frequency ablation instrument existing in the prior art, no effective solution has been brought up so far.

### SUMMARY OF THE DISCLOSURE

### Technical problem

In view of this, the present disclosure provides a radio-frequency ablation instrument and a control method and a control apparatus thereof, a system, an electronic device and a storage medium, to solve the problems of low accuracy of the output energy and poor stability in continuous output of the radio-frequency ablation instrument existing in the prior art.

### Technical solution

In one aspect, an embodiment of the present disclosure provides a control method of a radio-frequency ablation instrument, which includes acquiring a current ablation parameter; calculating a power value needed to be outputted in a next cycle according to the current ablation parameter; acquiring an input control voltage corresponding to the power value according to the power value needed to be outputted; and controlling the radio-frequency energy output according to the input control voltage.

In another aspect, an embodiment of the present disclosure provides a control apparatus of a radio-frequency ablation instrument, which includes a power calculation module, configured to acquire a current ablation parameter, and calculate a power value needed to be outputted by the radio-frequency ablation instrument in a next cycle according to the current ablation parameter; a voltage acquisition module, configured to acquire an input control voltage corresponding to the power value according to the power value needed to be outputted; and a radio-frequency output control module, configured to control the radio-frequency ablation instrument to output the radio-frequency energy according to the input control voltage.

In a further aspect, an embodiment of the present disclosure provides an electronic device, which includes a processor, and a memory configured to store instructions executable by the processor, wherein the processor is configured to perform the control method of a radio-frequency ablation instrument provided in the above embodiment by implementing the executable instructions.

In a further aspect, an embodiment of the present disclosure provides a radio-frequency ablation instrument, which includes an ablation parameter detection apparatus, configured to acquire a current ablation parameter in real time; a radio-frequency output apparatus; and a control apparatus of the radio-frequency ablation instrument provided in the above embodiments or an electronic device provided in the above embodiments.

In a further aspect, an embodiment of the present disclosure provides a radio-frequency ablation system, which includes an ablation apparatus; and a radio-frequency ablation instrument provided in the above embodiment, wherein the ablation apparatus is electrically connected to the radio-frequency ablation instrument, the radio-frequency ablation instrument is configured to provide radio-frequency energy, and the ablation apparatus is configured to deliver the radio-frequency energy to a to-be-ablated site.

In a further aspect, an embodiment of the present disclosure provides a computer readable storage medium, on which a computer program is stored, wherein when the computer program is executed by a processor, the control method of a radio-frequency ablation instrument provided in the above embodiment is performed.

### Beneficial effects

In various embodiments of the present disclosure, a power value needed to be outputted in a next cycle is calculated according to a current ablation parameter, an input control voltage corresponding to the power value is acquired according to the power value needed to be outputted, and the radio-frequency output apparatus is controlled to output the radio-frequency energy according to the input control voltage. Therefore, the radio-frequency ablation instrument can adjust the radio-frequency energy to be outputted in a next cycle according to the current ablation parameter. The above process is repeated for a period of time, during which radio-frequency energy is outputted to ablate the target object, such that accurate ablation of the target object is achieved by the radio-frequency ablation instrument. In this way, the output accuracy and stability in continuous output of the radio-frequency ablation instrument are improved, and the stability of the radio-frequency ablation instrument can also reach a certain degree of stability even when the output is disturbed, thus effectively ensuring the ablation effect.

### BRIEF DESCRIPTION OF DRAWINGS

To describe the technical solutions in the embodiments of the present disclosure or in the prior art more clearly and fully, the drawings to be used in the embodiments or in the prior art will be described briefly below. Apparently, the drawings in the following descriptions only show some embodiments of the present disclosure. Other drawings can be obtained by an ordinary one skilled in the art from these accompanying drawings without creative efforts.
Fig. 1 is a schematic diagram of a radio-frequency ablation instrument according to an embodiment of the present disclosure.
Fig. 2 is a schematic diagram showing an ablation parameter detection apparatus and a control apparatus according to an embodiment of the present disclosure.
Fig. 3 is a schematic diagram showing a radio-frequency ablation instrument according to another embodiment of the present disclosure.
Fig. 4 is a schematic diagram showing a human-machine interaction apparatus according to an embodiment of the present disclosure.
Fig. 5 is a schematic diagram showing an ablation interface of a radio-frequency ablation instrument according to an embodiment of the present disclosure.
Fig. 6 is a schematic diagram showing a parameter setting interface of a radio-frequency ablation instrument according to an embodiment of the present disclosure.
Fig. 7 is a schematic structural view of a mechanical button of a radio-frequency ablation instrument according to an embodiment of the present disclosure.
Fig. 8 is a schematic diagram showing a power calculation module according to an embodiment of the present disclosure.
Fig. 9 illustrates a correspondence relation between the output power of a radio-frequency output apparatus and the input control voltage according to an embodiment of the present disclosure.
Fig. 10 is a schematic diagram showing an ablation interface of a radio-frequency ablation instrument according to an embodiment of the present disclosure during an ablation process.
Fig. 11 is a schematic diagram showing an ablation interface of a radio-frequency ablation instrument according to an embodiment of the present disclosure during an ablation process.
Fig. 12 is a schematic flow chart of a control method of a radio-frequency ablation instrument according to an embodiment of the present disclosure.
Fig. 13 is a schematic flow chart of a control method of a radio-frequency ablation instrument according to an embodiment of the present disclosure.
Fig. 14 is a schematic flow chart of a control method of a radio-frequency ablation instrument according to an embodiment of the present disclosure.
Fig. 15 is a schematic structural diagram of a radio-frequency ablation system according to an embodiment of the present disclosure.
Fig. 16 is a schematic structural diagram of an electronic device according to an embodiment of the present disclosure.

### DDESCRIPTION OF EMBODIMENTS

To make the objects, technical solutions and advantages of the embodiments of the present disclosure clearer, the technical solutions according to the embodiments of the present disclosure will be described clearly and fully in combination with the accompanying drawings in the embodiments of the present disclosure. Apparently, the embodiments described are merely some, rather than all of the embodiments of the present disclosure. All other embodiments obtained by an ordinary one skilled in the art based on the embodiments of the present disclosure without creative efforts shall fall within the protection scope of the present disclosure.

Technical and scientific terms used herein have the same meaning as commonly understood by those skilled in the art to which the present disclosure belongs. As used in the present disclosure, Words "an" "a", "the", or the like do not indicate the limitation in number, but at least one. Words "Include", "comprise", or the like means that an element or object followed by the word covers an element or object following the word, without excluding other elements or objects. Words "connection", "connecting" or the like are not limited to physical or mechanical connection, but include electrical connection as well, whether they are direct or indirect.

Reference herein to "an embodiment" means that a particular feature, structure, or characteristic described in connection with the embodiment can be included in at least one embodiment of the present disclosure. The appearance of this phrase anywhere in the specification does not necessarily refer to the same embodiment, or a separate or alternative embodiment that is mutually exclusive with other embodiments. It is explicitly and implicitly understood by those skilled in the art that the embodiments described herein may be combined with other embodiments.

The term terminal involved in the embodiments of the present disclosure includes various handheld devices, on-board devices, wearable devices, computing devices or other processing devices connected to a wireless modem, and various forms of user equipment (UE), mobile stations (MS), and terminal devices etc having a display screen. For convenience of description, the devices mentioned above are collectively referred to as terminals.

The present disclosure claims priority of Chinese Patent Application No. 202011200579.8 filed with Intellectual Property Office of China on October 31, 2020 and entitled "Radio-frequency ablation instrument", the entire contents of which are hereby incorporated by reference.

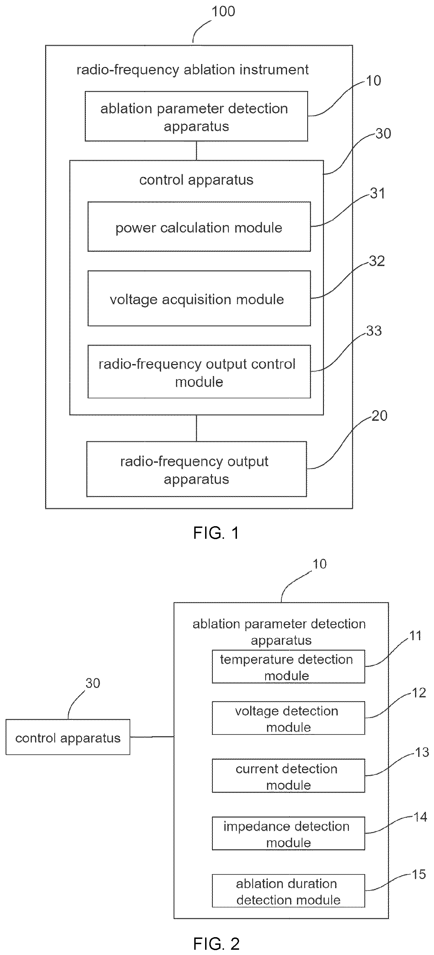

Referring to Fig. 1, which is a schematic diagram of a radio-frequency ablation instrument according to an embodiment of the present disclosure. The radio-frequency ablation instrument 100 is configured to ablate a target object. Specifically, the radio-frequency ablation instrument 100 includes an ablation parameter detection apparatus 10, a radio-frequency output apparatus 20 and a control apparatus 30. The control apparatus 30 is connected to the ablation parameter detection apparatus 10 and the radio-frequency output apparatus 20 respectively. The ablation parameter detection apparatus 10 is configured to acquire a current ablation parameter in real time. The control apparatus 30 includes a power calculation module 31, a voltage acquisition module 32 and a radio-frequency output control module 33. The power calculation module 31 is configured to calculate a power value needed to be outputted by the radio-frequency output apparatus 20 in a next cycle according to the current ablation parameter. The voltage acquisition module 32 is configured to acquire an input control voltage corresponding to the power value according to the power value needed to be outputted. The radio-frequency output control module 33 is configured to control the radio-frequency output apparatus 20 to output the radio-frequency energy according to the input control voltage.

The radio-frequency ablation instrument 100 of the present disclosure can calculate a power value needed to be outputted in a next cycle according to the current ablation parameter, acquire an input control voltage corresponding to the power value according to the power value needed to be outputted, and control the radio-frequency output apparatus 20 to output the radio-frequency energy according to the input control voltage. Therefore, the radio-frequency ablation instrument 100 can adjust the radio-frequency energy to be outputted in a next cycle according to the current ablation parameter. The above process is repeated for a period of time, during which radio-frequency energy is outputted to ablate the target object, such that accurate ablation of the target object is achieved by the radio-frequency ablation instrument 100. In this way, the output accuracy and stability in continuous output of the radio-frequency ablation instrument 100 are improved, and the stability of the radio-frequency ablation instrument 100 can also reach a certain degree of stability even when the output is disturbed, thus effectively ensuring the ablation effect.

Optionally, in one embodiment, referring to Fig. 2, which is a schematic diagram of an ablation parameter detection apparatus 10 according to an embodiment of the present disclosure, the ablation parameter detection apparatus 10 includes a temperature detection module 11, a voltage detection module 12 and a current detection module 13 respectively connected to the control apparatus 30. The temperature detection module 11 may be specifically a temperature detection device such as a temperature sensor, and configured to detect a current temperature of a target object. The voltage detection module 12 may be specifically a voltage detection device such as a voltage detector, and configured to detect a current voltage at an output terminal of the radio-frequency output apparatus 20. The current detection module 13 may be specifically a current detection device such as a current detector, and configured to detect a current electric current at the output terminal of the radio-frequency output apparatus 20.

Optionally, in one embodiment, referring to Fig. 2 again, the ablation parameter detection apparatus 10 further includes an impedance detection module 14 and ablation duration detection module 15 respectively connected to the control apparatus 30. The impedance detection module 14 may be specifically an impedance detection device such as an impedance detector, and configured to detect a current impedance in an ablation loop of the radio-frequency output apparatus 20. The ablation duration detection module 15 may be specifically an ablation duration detection device such as a timer, and configured to detect the duration of the ablation.

By means of the above modules, real-time parameters during ablation, for example, the current temperature, the current voltage, the current electric current, the current impedance and ablation duration, can be continuously detected in real time to know the status of ablation in real time.

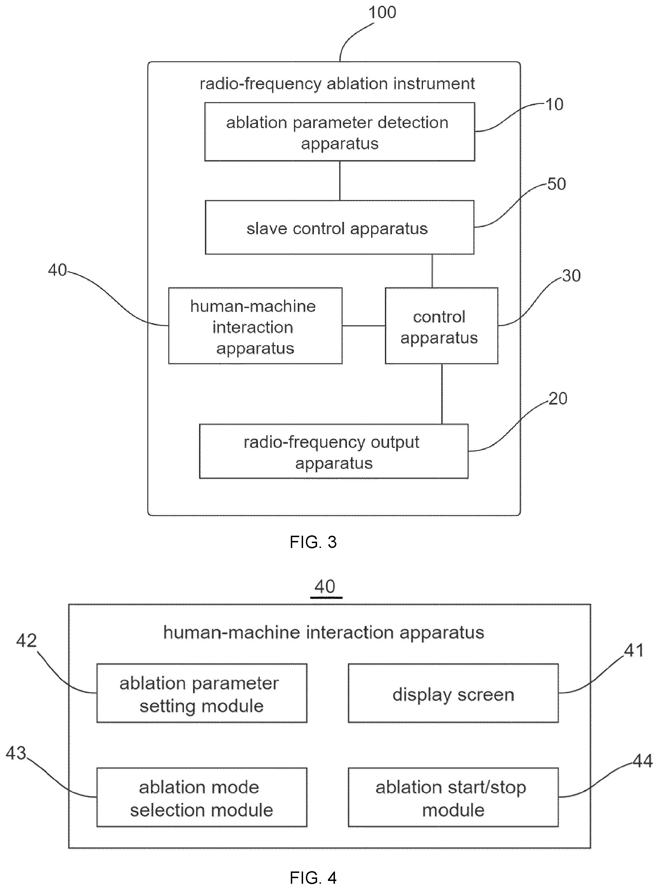

Optionally, in one embodiment, referring to Fig. 3, which is a schematic diagram of a radio-frequency ablation instrument 100 according to another embodiment of the present disclosure, different from the above embodiment, the radio-frequency ablation instrument 100 further includes a human-machine interaction apparatus 40 connected to the control apparatus 30. It can be understood that the human-machine interaction apparatus 40 may be, but is not limited to, a touch display screen, a mechanical button, a voice input device, and the like.

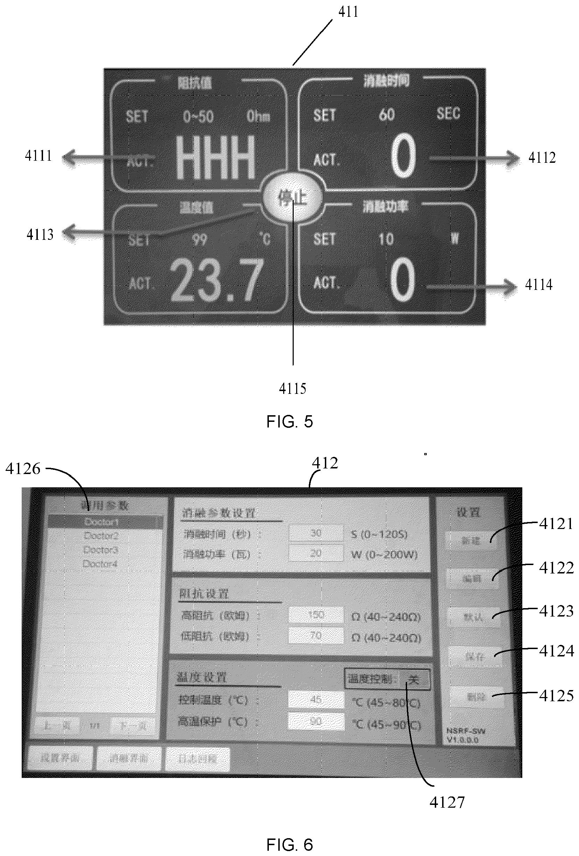

Optionally, in one embodiment, referring to Fig. 4, which is a schematic diagram of a human-machine interaction apparatus 40 according to an embodiment of the present disclosure, the human-machine interaction apparatus 40 includes a display screen 41 that is electrically connected to the control apparatus 30. When the radio-frequency ablation instrument 100 is powered on and started up, the control apparatus 30 controls the display screen 41 to display an ablation interface, for example, the ablation interface 411 as shown in Fig. 5. The ablation interface 411 is configured to display at least one of the following: a preset ablation parameter and a current ablation parameter. The preset ablation parameter includes at least one of the following parameters: an impedance, an ablation duration, a temperature and an ablation power. The current ablation parameter includes at least one of the following: an ablation impedance detected by the impedance detection module 14, an ablation duration detected by the ablation duration detection module 15, a real-time temperature detected by the temperature detection module 11, a current voltage detected by the voltage detection module 12, a current electric current detected by the current detection module 13, and a product of the current voltage times the current electric current, that is, a real-time ablation power. Specifically, in this embodiment, the ablation interface 411 can be divided into an impedance display area 4111, an ablation duration display area 4112, a temperature display area 4113 and an ablation power display area 4114. Each display area is configured to display a preset corresponding ablation parameter and a current corresponding ablation parameter. That is, the impedance display area 4111 is configured to display the preset impedance and the current impedance; the ablation duration display area 4112 is configured to display the preset ablation duration and the current elapsed ablation duration; the temperature display area 4113 is configured to display the preset temperature and the current temperature; and the ablation power display area 4114 is configured to display the preset ablation power and the current ablation power.

Optionally, in one of the embodiment, referring to Fig. 4 again, the human-machine interaction apparatus 40 further includes an ablation parameter setting module 42. The ablation parameter setting module 42 is configured to generate, in response to a human-machine interaction operation for setting the ablation parameter, a corresponding ablation parameter setting control signal. The control apparatus 30 is configured to set an ablation parameter according to the ablation parameter setting control signal. Specifically, the parameter needed to be set includes at least one of an impedance range, an ablation voltage range, an ablation current range, an ablation duration and a temperature protection range. For example, as shown in Fig. 6, the control apparatus 30 controls the display screen 41 to display a parameter setting interface 412. The parameter setting interface 412 includes a create function key 4121, an edit function key 4122, a default function key 4123, a save function key 4124, a delete function key 4125 and an invocation parameter display area 4126. The create function key 4121 is configured to create a set of ablation parameters, the edit function key 4122 is configured to edit a selected set of ablation parameters, the default function key 4123 is configured to reset each parameter to a default value, the save function key 4124 is configured to save a selected set of ablation parameters, the delete function key 4125 is configured to delete a selected set of ablation parameters, and the invocation parameter display area 4126 is configured to display at least one set ablation parameter, for example, Doctor 1, Doctor 2, Doctor 3, and on the like. When a set of ablation parameters is created or edited, the ablation parameters, for example, the temperature, ablation duration, ablation power, ablation impedance or the like may be created or edited. It should be noted that the setting of each ablation parameter needs to meet a corresponding parameter range. For example, the ablation duration is required to be within 0-120 sec, the ablation power is required to be within 0-200 W, or the like.

Optionally, in one embodiment, referring to Figs. 5 and 6, the display screen 41 is a touch display screen. Specifically, when respective input boxes corresponding to the ablation time, ablation power, high impedance, low impedance, control temperature, and high temperature protection on the parameter setting interface 412 are touched, a soft keyboard will pop up on the parameter setting interface 412, for entering and saving a corresponding value range by a user. That is, the function of the ablation parameter setting module 42 can be implemented by a touch operation on the touch display screen.

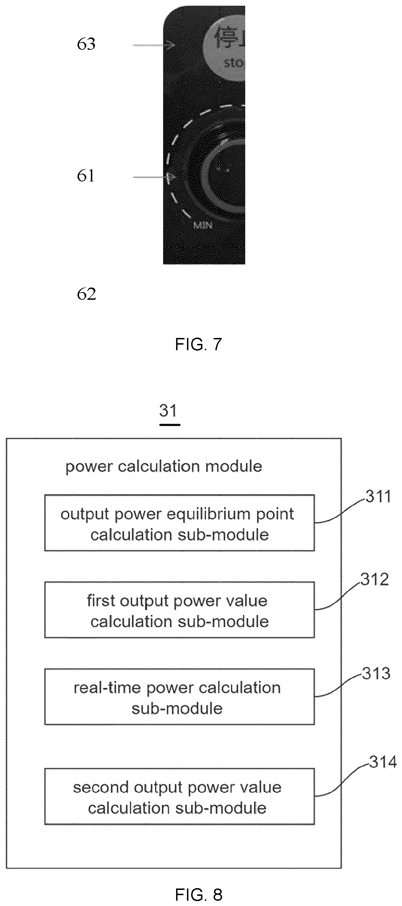

Optionally, in one embodiment, referring to Fig. 7, a plurality of mechanical buttons are provided on the radio-frequency ablation instrument 100, and the mechanical buttons are arranged adjacent to the display screen 41, or arranged at other suitable positions on the radio-frequency ablation instrument 100. The mechanical buttons include an adjustment knob 61. When the display screen 41 displays the parameter setting interface 412, the adjustment knob 61 can be rotated to move the cursor on the parameter setting interface 412 to the input box corresponding to one of the ablation time, ablation power, high impedance, low impedance, control temperature, and high temperature protection. The adjustment knob 61 can be rotated to adjust the parameter setting in the input box. After the parameter adjustment, the data can be saved by pressing the adjustment knob 61. Namely, the function of the ablation parameter setting module 42 can also be implemented by the adjustment knob 61.

Optionally, in one the embodiment, referring to Fig. 4 again, the ablation mode of the radio-frequency ablation instrument 100 includes a constant-temperature ablation mode and a constant-power ablation mode. The constant-temperature ablation mode means that the radio-frequency energy output is controlled to achieve a constant temperature, that is, the radio-frequency energy output is controlled to control the temperature at the target object to be maintained constant over a continuous period of time. Therefore, the control of the radio-frequency energy output with constant temperature mainly functions to control the radio-frequency energy receiving point at the target object to receive the radio-frequency energy while maintaining a constant temperature, so that the target object requiring receiving the radio-frequency energy to maintain a constant temperature can be ablated more accurately and effectively. The constant-power ablation mode means that the radio-frequency energy output is controlled to have a constant power, that is, the radio-frequency energy output is controlled to control the power received at the target object to be maintained constant over a continuous period of time. Therefore, the control of the radio-frequency energy output with constant power mainly functions to guarantee that the target object requiring constant energy in a continuous period of time can receive sufficient energy, so that the target object can be ablated more accurately and effectively. Referring to Fig. 4 again, the human-machine interaction apparatus 40 further includes an ablation mode selection module 43. The ablation mode selection module 43 generates, in response to a human-machine interaction operation for selecting an ablation mode, a corresponding ablation mode selection signal, and the control apparatus 30 selects, in response to the ablation mode selection signal, one of the constant-temperature ablation mode and the constant-power ablation mode to perform ablation.

Specifically, referring to Fig. 6, in one embodiment, the ablation mode selection module 43 is a virtual key 4127 on the parameter setting interface 412. When the virtual key 4127 is touched and shows that the temperature control is off, the constant-power mode is enabled; and when the virtual key 4127 is touched and shows that the temperature control is on, the constant-temperature mode is enabled.

Optionally, in other embodiments, the ablation mode selection module 43 may be one of the plurality of mechanical buttons, which can be pressed or rotated to achieve the selection of the ablation mode.

Optionally, in one embodiment, the mechanical button of the ablation mode selection module 43 and the adjustment knob 61 of the ablation parameter setting module 42 are formed as a same mechanical button. When the display screen 41 displays the parameter setting interface 412, the adjustment knob 61 can be rotated to move the cursor on the parameter setting interface 412 to the virtual key 4127. At that time, the adjustment knob 61 can be rotated or pressed to achieve the selection of the ablation mode.

Therefore, in the present disclosure, the constant-temperature ablation mode or constant-power ablation mode can be selected according to different application requirements. Optimization is performed for different application requirements, to achieve the accurate control of the output energy from the radio-frequency ablation instrument 100.

When one of the ablation modes is selected to perform the ablation, a corresponding parameter in this ablation mode is set. Of course, it can be understood that the parameter needed to be set in the constant-temperature ablation mode is different from the parameter needed to be set in the constant-power ablation mode. Specifically, the parameter needed to be set in the constant-temperature ablation mode includes at least a preset constant-temperature point and a preset output power. Optionally, in one embodiment, the parameter needed to be set in the constant-temperature ablation mode further at least includes one of the following: an impedance range, an ablation voltage range, an ablation current range, and an ablation duration. The range of the preset constant-temperature point includes, but is not limited to, 0-100°C, the range of the preset output power includes, but is not limited to, 0-200 W. The impedance range includes, but is not limited to, 0-600 Ω. The ablation voltage range includes, but is not limited to, 0-220 V. The ablation current range includes, but is not limited to, 0-5 A. The ablation duration includes, but is not limited to, 0-300 s. The parameter needed to be set in the constant-power ablation mode includes at least a preset constant-ablation power point. Optionally, in one embodiment, the parameter needed to be set in the constant-power ablation mode further at least includes one of the following: an impedance range, an ablation voltage range, an ablation current range, an ablation duration, and a temperature protection range. The range of the preset constant-ablation power point includes, but is not limited to, 0-200 W. The impedance range includes, but is not limited to, 0-600 Ω. The ablation voltage range includes, but is not limited to, 0-220 V. The ablation current range includes, but is not limited to, 0-5 A. The ablation duration includes, but is not limited to, 0-300 s. The temperature protection range includes, but is not limited to, 0-100°C. It should be further noted that the range of the parameter needed to be set in the constant-temperature ablation mode and the constant-power ablation mode can be adaptively adjusted according to practical applications.

It should be further noted that the calculation process of the parameters in the constant-temperature ablation mode and the constant-power ablation mode may be different.

First, in the constant-temperature ablation mode, the power value needed to be outputted by the radio-frequency output apparatus 20 in a next cycle is calculated through a process as follows.

Referring to Fig. 8, which is a schematic diagram of a power calculation module according to an embodiment of the present disclosure. In the constant-temperature ablation mode, the power calculation module 31 includes an output power equilibrium point calculation sub-module 311 and a first output power value calculation sub-module 312. The output power equilibrium point calculation sub-module 311 is configured to calculate an output power equilibrium point of the radio-frequency output apparatus 20 in a next cycle according to the current temperature and the preset constant-temperature point. The first output power value calculation sub-module 312 is configured to calculate a power value needed to be outputted by the radio-frequency output apparatus 20 in the next cycle according to the current voltage, the current electric current, and the output power equilibrium point in the next cycle.

Optionally, in one embodiment, the output power equilibrium point calculation sub-module 311 is specifically configured to
calculate an output power equilibrium point of the radio-frequency output apparatus 20 meeting the constant temperature in a next cycle by an incremental algorithm according to the current temperature and the preset constant-temperature point.

Optionally, in one embodiment, a specific process of calculating, by the output power equilibrium point calculation sub-module, an output power equilibrium point of the radio-frequency output apparatus 20 meeting the constant temperature in a next cycle by an incremental algorithm includes
calculating a first deviation, wherein the first deviation = the preset constant-temperature point - the current temperature;
obtaining an output power compensation value by querying the incremental level according to the first deviation; and
calculating the output power equilibrium point meeting constant temperature in the next cycle according to the preset output power and the output power compensation value, wherein the output power equilibrium point in the next cycle = the preset output power + the output power compensation value.

The incremental algorithm is to calculate the difference between the actual value and the preset value, the larger the difference is, the higher the compensation value will be, and the smaller the difference is, the lower the compensation value will be. The incremental level is obtained by empirical value, for example, 0.1, 0.2, 0.5, 1, 2, 5, and 10. If a rapid response is required, the incremental value in the incremental level is increased; and if smoothly approaching is required, the incremental value in the incremental level is appropriately lowered. In this embodiment, the difference between the current temperature and the preset constant-temperature point is obtained to obtain the first deviation, and then the compensation is performed by an incremental algorithm according to the first deviation.

For example, in one embodiment, the preset constant-temperature point is 50°C, the current temperature is 30°C, the incremental level is 10, and the preset output power is 30 W. Then a specific process of calculating an output power equilibrium point of the radio-frequency output apparatus 20 meeting the constant temperature in a next cycle includes
calculating a first deviation, wherein the first deviation = the preset constant-temperature point(50°C) - the current temperature(30°C) = 20°C;
querying a compensation value (12 W) from a table of incremental level (10) according to the first deviation (20°C); and
obtaining the output power equilibrium point in the next cycle, wherein the output power equilibrium point in the next cycle = the preset output power (30 W) + the compensation value (12 W) = 42 W.

Optionally, in one embodiment, the first output power value calculation sub-module 312 is specifically configured to
calculate a real-time power according to the acquired current voltage and current electric current, wherein the real-time power is a product of the current voltage and the current electric current; and
calculate the power value needed to be outputted by the radio-frequency output apparatus 20 in the next cycle with the PID algorithm according to the real-time power and the output power equilibrium point in the next cycle.

Optionally, in one embodiment, the step of calculating, by the first output power value calculation sub-module 312, the power value needed to be outputted by the radio-frequency output apparatus 20 in the next cycle with the PID algorithm according to the real-time power and the output power equilibrium point in the next cycle includes specifically:
calculating a second deviation, wherein the second deviation = the output power equilibrium point in the next cycle - the real-time power, for example, the second deviation = the output power equilibrium point (42 W) in the next cycle - the real-time power (28 W) = 14 W;
calculating a first P deviation, wherein the first P deviation = the second deviation - a second deviation in a previous cycle;
calculating a first I deviation, wherein the first I deviation = the second deviation;
calculating a first D deviation, wherein the first D deviation = the second deviation - 2* the second deviation in the previous cycle + a second deviation in a further previous cycle which is a preceding cycle adjacent to and before the previous cycle;
calculating a power compensation value in the next cycle, wherein the power compensation value in the next cycle = P coefficient * the first P deviation + I coefficient * the first I deviation + D coefficient * the first D deviation; and
calculating the power value needed to be outputted in the next cycle, wherein the power value needed to be outputted in the next cycle = the real-time power + the power compensation value in the next cycle;
wherein the P coefficient, I coefficient, and D coefficient are values obtained from test data or empirical values.

For example, in one embodiment, the P coefficient is in the range of 0-100, the I coefficient is in the range of 0-100, and the D coefficient is in the range of 0-100.

Therefore, a deviation is effectively corrected by a compensation value which is calculated by an algorithm using the first P deviation, the first I deviation, and the first D deviation according to the deviation between the output power equilibrium point in the next cycle and the real-time power, to achieve the dynamic balance of a target. The first P deviation responds to the system deviation, so whenever there is a deviation, the proportion works. The first I deviation mainly serves to eliminate the static difference, and the static difference is the difference between the input and output after the system is steady. The first I deviation is to offset the static difference of the system through the accumulation of deviation. The first D deviation responds to the change trend of the deviation, and adjustment can be performed in advance according to the change trend of the first D deviation, so as to increase the response speed.

In the constant-power ablation mode, a process of calculating a power value needed to be outputted by the radio-frequency output apparatus 20 in a next cycle is specifically described as follows.

Referring to Fig. 8 again, in the constant-power ablation mode, the power calculation module 31 further includes a real-time power calculation sub-module 313 and a second output power value calculation sub-module 314.

The real-time power calculation sub-module 313 is configured to calculate a real-time power of the target object according to the current voltage and the current electric current, wherein the real-time power is a product of the current voltage and the current electric current; and
the second output power value calculation sub-module 314 is configured to calculate the power value needed to be outputted by the radio-frequency output apparatus 20 in the next cycle with the PID algorithm according to the real-time power and the preset constant-ablation power point.

Optionally, in one embodiment, the step of calculating, by the second output power value calculation sub-module 314, the power value needed to be outputted by the radio-frequency output apparatus 20 in the next cycle with the PID algorithm according to the real-time power and the preset constant-ablation power point, includes:
calculating a third deviation, wherein the third deviation = the preset constant-ablation power point - the real-time power;
calculating a second P deviation, wherein the second P deviation = the third deviation - a third deviation in a previous cycle;
calculating a second I deviation, wherein the second I deviation = the third deviation;
calculating a second D deviation, wherein the second D deviation = the third deviation -2* the third deviation in the previous cycle + a third deviation in a further previous cycle which is a preceding cycle adjacent to and before the previous cycle;
calculating a next output point compensation power value, wherein the next output point compensation power value =P coefficient * the second P deviation + I coefficient * the second I deviation + D coefficient * the second D deviation; and
calculating the power value needed to be outputted in the next cycle, wherein the power value needed to be outputted in the next cycle = the real-time power + the next output point compensation power value;
wherein the P coefficient, I coefficient, and D coefficient are values obtained from test data or empirical values. For example, in one embodiment, the P coefficient is in the range of 0-100, the I coefficient is in the range of 0-100, and the D coefficient is in the range of 0-100.

Therefore, a deviation is effectively corrected by a compensation value which is calculated by an algorithm using the second P deviation, the second I deviation, and the second D deviation according to the deviation between the preset constant-ablation power point and the real-time power, to achieve the dynamic balance of a target. The second P deviation responds to the system deviation, so whenever there is a deviation, the proportion works. The second I deviation mainly serves to eliminate the static difference, and the static difference is the difference between the input and output after the system is steady. The second I deviation is to offset the static difference of the system through the accumulation of deviation. The second D deviation responds to the change trend of the deviation, and adjustment can be performed in advance according to the change trend of the second D deviation, so as to increase the response speed.

Therefore, in the present disclosure, the constant-temperature ablation mode or constant-power ablation mode can be selected according to requirements of the target object, so the present disclosure has a wider range of application. Moreover, the incremental algorithm and PID algorithm are employed to control the real-time ablation energy output, which increases the real-time stability, and guarantees that the system is accurate and effective in both modes. Therefore, the real-time stability, safety, accuracy, stability, and practicality are greatly improved.

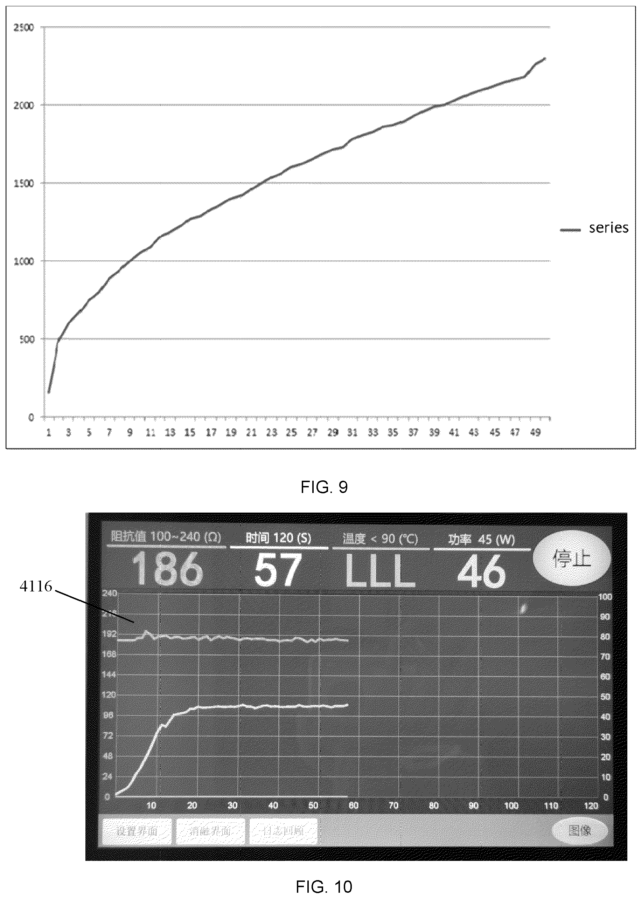

Optionally, in one embodiment, referring to Fig. 9, which shows a correspondence relation between the output power of a radio-frequency output apparatus 20 and the input control voltage according to an embodiment of the present disclosure. The radio-frequency output apparatus 20 is integrated with a radio-frequency output related function, having an output power that is controlled by inputting an input control voltage corresponding to the output power, wherein the correspondence relation between the input control voltage and the output power is nonlinear. The horizontal coordinate in Fig. 9 represents the output power of the radio-frequency output apparatus 20, the vertical ordinate represents the input control voltage of the radio-frequency output apparatus 20, and the curve represents the relation between the input control voltage and corresponding power of the radio-frequency output apparatus 20. The correspondence relation shown by the curve gives values obtained from test data or empirical values. For example, if the output power is 10 W, the corresponding input control voltage of the radio-frequency output apparatus 20 is 1050 V; and if the output power is 50 W, the corresponding input control voltage of the radio-frequency output apparatus 20 is 2300 V. Therefore, the control apparatus 30 obtains an input control voltage corresponding to the output power from a correspondence relation table of the output power vs the input control voltage according to the output power of the radio-frequency output apparatus 20. This further improves the output accuracy and stability in continuous output of the radio-frequency ablation instrument 100.

Optionally, in one embodiment, referring to Fig. 4 again, the human-machine interaction apparatus 40 further includes an ablation start/stop module 44. The ablation start/stop module 44 is configured to generate, in response to a human-machine interaction operation for starting/stopping ablation, a corresponding ablation start/stop control signal. The control apparatus 30 is configured to start the working of the radio-frequency ablation instrument 100 or stop the working of the radio-frequency ablation instrument 100 in response to the ablation start/stop control signal.

Optionally, in one embodiment, referring to Fig. 5 again, the control apparatus 30 controls the ablation interface 411 displayed on the display screen 41 to further show a virtual key 4115, and the virtual key 4115 is configured to start or stop the ablation. Namely, the function of the ablation start/stop module 44 may be implemented by the virtual key 4115. When the control apparatus 30 controls the display screen 41 to display the ablation interface 411, the virtual key 4115 generates, in response to a selection operation of a user, a start control signal, and the control apparatus 30 is configured to start, in response to the start control signal, the working of the radio-frequency ablation instrument 100. After the ablation is started, the virtual key 4115 also generates, in response to a selection operation of a user, a stop control signal, and the control apparatus 30 is configured to stop, in response to the stop control signal, the working of the radio-frequency ablation instrument 100.

Optionally, in another embodiment, referring to Fig. 7, the mechanical buttons include a start button 62 and a stop button 63. The start button 62 is configured to start the working of the radio-frequency ablation instrument100, and the stop button 63 is configured to stop the working of the radio-frequency ablation instrument 100. Namely, the function of the ablation start/stop module 44 can also be implemented by the start button 62 and the stop button 63.

Optionally, in another embodiment, the radio-frequency ablation instrument 100 includes a foot switch. When the foot switch is stepped down, the ablation is started, and the radio-frequency ablation instrument 100 starts to output the radio-frequency energy.

Optionally, in another embodiment, the foot switch needs to be persistently stepped down during the ablation process. Once the foot switch is released, the ablation is stopped, and accordingly the radio-frequency ablation instrument 100 stops outputting the radio-frequency energy.

Optionally, in another embodiment, if the set ablation power and/or the set ablation duration is/are 0, the ablation cannot be started when the foot switch is stepped down.

Optionally, in another embodiment, if the impedance is lower than the set minimum impedance protection value or higher than the set maximum impedance protection value, the ablation cannot be started when the foot switch is stepped down.

Optionally, in another embodiment, after the ablation is started by stepping down the foot switch, the ablation can also be stopped by pressing the stop button 63. Similarly, after the ablation is started by pressing the start button 62, the ablation can also be stopped by releasing the foot switch.

Optionally, in one embodiment, during the radio-frequency ablation process implemented by the radio-frequency output apparatus 20 , the control apparatus 30 controls the radio-frequency output apparatus 20 to stop ablation or adjust the radio-frequency energy output if one of the following events occur: the current impedance goes beyond the impedance range, the current voltage goes beyond the ablation voltage range, the current electric current goes beyond the ablation current range, the ablation duration goes beyond the set ablation duration, and the current temperature goes beyond the temperature protection range.

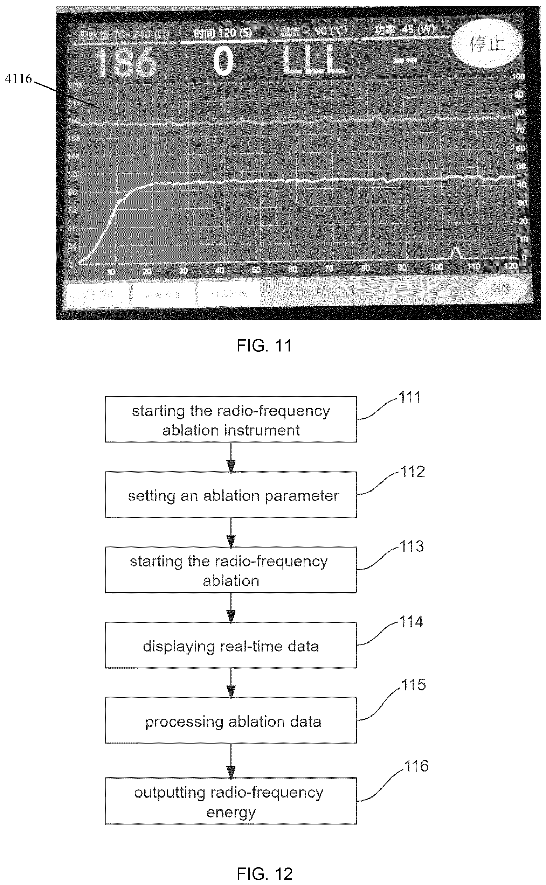

Optionally, in one embodiment, referring to Figs. 10 and 11, the ablation interface 411 is further configured to display a waveform pattern 4116 of the current ablation parameter during the ablation. Further, the set ablation parameter and the current ablation parameter are displayed at the top of the ablation interface 411 during the ablation process, and the waveform pattern 4116 is displayed in a lower region of the ablation interface 411. It can be understood that the three curves in the waveform pattern 4116 are respectively, from the top to the bottom, an impedance fluctuation curve, an ablation power fluctuation curve and a temperature fluctuation curve. The three curves extend rightwards with the elapse of the ablation time, until the ablation is completed.

Optionally, in one embodiment, referring to Fig. 3 again, the radio-frequency ablation instrument 100 further includes a slave control apparatus 50. The slave control apparatus 50 is configured to control the ablation parameter detection apparatus 10 to acquire the current ablation parameter in real time, and the control apparatus 30 is configured to obtain the detected current ablation parameter from the slave control apparatus 50.

Therefore, the slave control apparatus 50 is utilized to acquire and process the ablation parameter of the target object in real time, and the control apparatus 30 then performs radio-frequency ablation control according to the processed real-time data. In this way, the work is implemented by two processors, and when a fault occurs, the fault can be identified in time, so as to ensure the safety and reliability of the apparatus during use.

Fig. 12 is a schematic flow chart of a control method of a radio-frequency ablation instrument 100 according to an embodiment of the present disclosure. The structure and function of the radio-frequency ablation instrument 100 are specifically as shown in Figs. 1 to 11. The sequence of implementing the control method is not limited to the sequence shown in Fig. 12, and adjustments may be made according to actual needs. Specifically, in this embodiment, the control method of the radio-frequency ablation instrument 100 includes:
Step 111: starting the radio-frequency ablation instrument 100.
   Specifically, in one embodiment, after the radio-frequency ablation instrument 100 is started, the radio-frequency ablation instrument 100 is initialized, to complete the preparation of the radio-frequency ablation instrument 100.
Step 112: setting an ablation parameter.

Specifically, in one embodiment, the control apparatus 30 controls the display screen 41 to display a parameter setting interface 412. The ablation parameter setting module 42 generates, in response to a human-machine interaction operation for setting an ablation parameter on the parameter setting interface 412, a corresponding ablation parameter setting control signal. The control apparatus 30 sets a rational ablation parameter for a particular target object according to the ablation parameter setting control signal. Specifically, the parameter needed to be set includes at least one of the following: a preset constant-temperature point, a preset output power, a preset constant-ablation power point, an impedance range, an ablation voltage range, an ablation current range, an ablation duration and a temperature protection range.

Optionally, in one embodiment, referring to Figs. 5 and 6, when respective input boxes corresponding to the ablation time, ablation power, high impedance, low impedance, control temperature, and high temperature protection on the parameter setting interface 412 are touched, a soft keyboard will pop up on the parameter setting interface 412, for inputting a corresponding value range by a user and saving the same. Namely, the function of the ablation parameter setting module 42 can be implemented by a touch operation on the touch display screen.

Optionally in one embodiment, referring to Fig. 7, when the display screen 41 displays the parameter setting interface 412, the adjustment knob 61 can be rotated to move the cursor on the parameter setting interface 412 to the input box corresponding to one of the ablation time, ablation power, high impedance, low impedance, control temperature, and high temperature protection. The adjustment knob 61 can be rotated to adjust the parameter setting in the input box. After the parameter adjustment, the adjustment knob 61 can be pressed to save the data. Namely, the function of the ablation parameter setting module 42 can also be implemented by the adjustment knob 61.

Step 113: starting the radio-frequency ablation.

Specifically, in one embodiment, referring to Fig. 5, the control apparatus 30 controls the display screen 41 to display an ablation interface 411, and a virtual key 4115 is displayed on the ablation interface 411, wherein the virtual key 4115 generates, in response to a selection operation of a user, a start control signal, and the control apparatus 30 is configured to start, in response to the start control signal, the working of the radio-frequency ablation instrument 100.

Optionally, in another embodiment, referring to Fig. 7, the start button 62 generates, in response to a selection operation of a user, a start control signal, and the control apparatus 30 is configured to start, in response to the start control signal, the working of the radio-frequency ablation instrument 100.

Optionally, in another embodiment, the radio-frequency ablation instrument 100 includes a foot switch. By stepping down the foot switch, the ablation is started or stopped, and the radio-frequency ablation instrument 100 is controlled to output the radio-frequency energy.

Step 114: displaying real-time data.

Specifically, in one embodiment, the control apparatus 30 controls to display the set ablation parameter, the current ablation parameter and the waveform pattern 4116 of the current ablation parameter during the ablation on the ablation interface 411.

Step 115: processing ablation data.

Specifically, in one embodiment, the control apparatus 30 calculates a power value needed to be outputted by the radio-frequency output apparatus 20 in a next cycle according to the current ablation parameter and acquires an input control voltage corresponding to the power value according to the power value needed to be outputted.

Step 116: outputting radio-frequency energy.

Specifically, in one embodiment, the control apparatus 30 controls the radio-frequency output apparatus 20 to output the radio-frequency energy according to the input control voltage.

A specific process of implementing the above steps by the control apparatus 30 can also be made reference to the relevant descriptions in the embodiments shown in Figs. 1 to 11.

Therefore, the radio-frequency ablation instrument 100 can adjust the radio-frequency energy to be outputted in a next cycle according to the current ablation parameter. The above process is repeated for a period of time, during which radio-frequency energy is outputted to ablate the target object, such that accurate ablation of the target object is achieved by the radio-frequency ablation instrument 100. In this way, the output accuracy and stability in continuous output of the radio-frequency ablation instrument 100 are improved, and the stability of the radio-frequency ablation instrument 100 can also reach a certain degree of stability even when the output is disturbed, thus effectively ensuring the ablation effect.

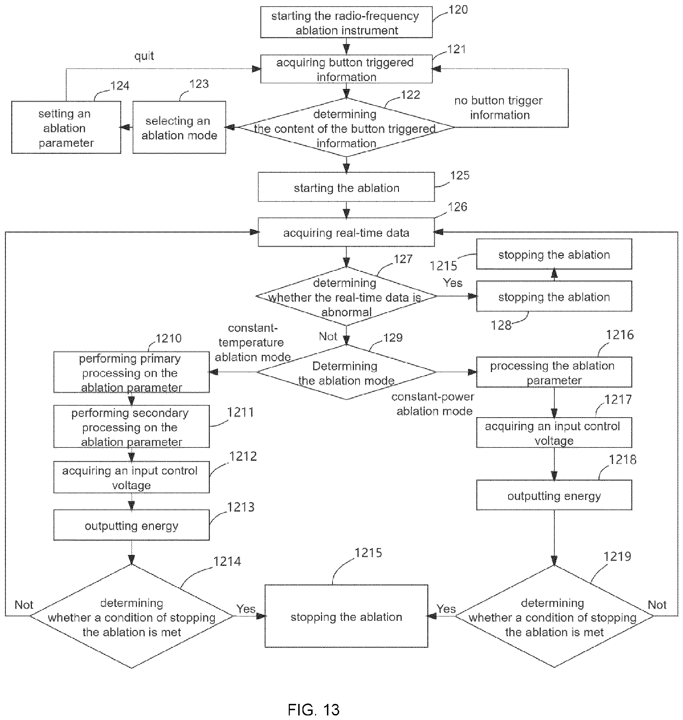

Fig. 13 is a schematic flow chart of a control method of a radio-frequency ablation instrument 100 according to an embodiment of the present disclosure. The structure and function of the radio-frequency ablation instrument 100 are specifically as shown in Figs. 1 to 11. The sequence of implementing the control method is not limited to the sequence shown in Fig. 13, and adjustments may be made according to actual needs. Specifically, in this embodiment, the control method of the radio-frequency ablation instrument 100 includes:
Step 120: starting the radio-frequency ablation instrument 100.
   Specifically, in one embodiment, after the radio-frequency ablation instrument 100 is started, the radio-frequency ablation instrument 100 is initialized, to complete the preparation of the radio-frequency ablation instrument 100.
Step 121: acquiring button triggered information.
Step 122: determining the content of the button triggered information. If the button triggered information is to select an ablation mode, Step 123 is performed. If the button triggered information is to start the ablation, Step 125 is performed. If there is no button triggered information, Step 121 is repeated.
Step 123: selecting an ablation mode.

The ablation mode selection module 43 generates, in response to a human-machine interaction operation for selecting an ablation mode, a corresponding ablation mode selection signal, and the control apparatus 30 selects, in response to the ablation mode selection signal, one of the constant-temperature ablation mode and the constant-power ablation mode.

Specifically, referring to Fig. 6, in this embodiment, the ablation mode selection module 43 is a virtual key 4127 on the parameter setting interface 412. When the virtual key 4127 is touched and shows that the temperature control is off, the constant-power mode is enabled; and when the virtual key 4127 is touched and shows that the temperature control is on, the constant-temperature mode is enabled.

Optionally, in other embodiments, the ablation mode selection module 43 may be one of the mechanical buttons, which can be pressed or rotated to achieve the selection of the ablation mode.

Optionally, in one embodiment, the mechanical button of the ablation mode selection module 43 and the adjustment knob 61 of the ablation parameter setting module 42 are formed as a same mechanical button. When the display screen 41 displays the parameter setting interface 412, the adjustment knob 61 can be rotated to move the cursor on the parameter setting interface 412 to the virtual key 4127. In this case, the adjustment knob 61 can be rotated or pressed to achieve the selection of the ablation mode.

Step 124: setting an ablation parameter.

Specifically, in one embodiment, the control apparatus 30 controls the display screen 41 to display a parameter setting interface 412. The ablation parameter setting module 42 generates, in response to a human-machine interaction operation for setting an ablation parameter on the parameter setting interface 412, a corresponding ablation parameter setting control signal. The control apparatus 30 sets a rational ablation parameter for a particular target object according to the ablation parameter setting control signal. Specifically, the parameter needed to be set includes at least one of the following: a preset constant-temperature point, a preset output power, a preset constant-ablation power point, an impedance range, an ablation voltage range, an ablation current range, an ablation duration and a temperature protection range.

Specifically, the parameter needed to be set in the constant-temperature ablation mode includes at least a preset constant-temperature point and a preset output power. Optionally, in one embodiment, the parameter needed to be set in the constant-temperature ablation mode further includes at least one of an impedance range, an ablation voltage range, an ablation current range, and an ablation duration. Optionally, in one embodiment, the parameter needed to be set in the constant-power ablation mode further includes at least one of an impedance range, an ablation voltage range, an ablation current range, an ablation duration, and a temperature protection range.

Referring to Figs. 5 and 6, optionally, in one embodiment, when respective input boxes corresponding to the ablation time, ablation power, high impedance, low impedance, control temperature, and high temperature protection on the parameter setting interface 412 are touched, a soft keyboard will pop up on the parameter setting interface 412, for inputting a corresponding value range by a user and saving the same. Namely, the function of the ablation parameter setting module 42 can be implemented by a touch operation on the touch display screen.

Referring to Fig. 7, optionally in one embodiment, when the display screen 41 displays the parameter setting interface 412, the adjustment knob 61 can be rotated to move the cursor on the parameter setting interface 412 to the input box corresponding to one of the ablation time, ablation power, high impedance, low impedance, control temperature, and high temperature protection. The adjustment knob 61 can be rotated to adjust the parameter setting in the input box. After the parameter adjustment, the adjustment knob 61 can be pressed to save the data.

Step 125: starting the ablation.

Specifically, in one embodiment, the control apparatus 30 controls the display screen 41 to display an ablation interface 411, and a virtual key 4115 is displayed on the ablation interface 411. The virtual key 4115 generates, in response to a selection operation of a user, a start control signal, and the control apparatus 30 is configured to start, in response to the start control signal, the working of the radio-frequency ablation instrument 100.

Referring to Fig. 7, optionally, in another embodiment, the start button 62 generates, in response to a selection operation of a user, a start control signal, and the control apparatus 30 is configured to start, in response to the start control signal, the working of the radio-frequency ablation instrument 100.

Step 126: acquiring real-time data.

Specifically, in one embodiment, the slave control apparatus 50 controls the ablation parameter detection apparatus 10 to acquire a current ablation parameter in real time, and the control apparatus 30 obtains the detected current ablation parameter from the slave control apparatus 50. Specifically, in this embodiment, the current ablation parameter includes: a current temperature, a current voltage, a current electric current, a current impedance and an ablation duration. Further, in one embodiment, the ablation interface 411 further displays a waveform pattern 4116 of the current ablation parameter during the ablation.

Step 127: determining whether the real-time data is abnormal.

Specifically, in one embodiment, the control apparatus 30 determines whether the current impedance goes beyond the impedance range, whether the current voltage goes beyond the ablation voltage range, whether the current electric current goes beyond the ablation current range, whether the ablation duration goes beyond the set ablation duration and whether the current temperature goes beyond the temperature protection range. If the current impedance goes beyond the impedance range, the current voltage goes beyond the ablation voltage range, the current electric current goes beyond the ablation current range, the ablation duration goes beyond the set ablation duration or the current temperature goes beyond the temperature protection range, Step 128 is performed; and if not, Step 129 is performed. It should be noted that in other embodiments, if the real-time data is abnormal, the real-time data may be rendered normal by parameter adjustment.

Step 128: stopping the ablation.

Specifically, in one embodiment, the control apparatus 30 controls the radio-frequency output apparatus 20 to stop the ablation, and Step 1215 is performed.

Step 129: Determining the ablation mode.

The control apparatus 30 determines a currently selected ablation mode according to the ablation mode selection signal generated by the human-machine interaction operation. If it is determined that the ablation mode is the constant-temperature ablation mode, Step 1210 is performed. If it is determined that the ablation mode is the constant-power ablation mode, Step 1216 is performed.

Step 1210: performing primary processing on the ablation parameter.

In the constant-temperature ablation mode, the performing primary processing on the ablation parameter in one embodiment means that the output power equilibrium point calculation sub-module 311 calculates an output power equilibrium point of the radio-frequency output apparatus 20 in a next cycle according to the current temperature and the preset constant-temperature point.

Specifically, the output power equilibrium point of the radio-frequency output apparatus 20 meeting the constant temperature in a next cycle is calculated with an incremental algorithm according to the current temperature and the preset constant-temperature point.

Specifically, a specific process of calculating, by the output power equilibrium point calculation sub-module, an output power equilibrium point of the radio-frequency output apparatus 20 meeting the constant temperature in a next cycle with an incremental algorithm includes
calculating a first deviation, wherein the first deviation = the preset constant-temperature point - the current temperature;
obtaining an output power compensation value by querying the incremental level according to the first deviation; and
calculating the output power equilibrium point meeting constant temperature in the next cycle according to the preset output power and the output power compensation value, wherein the output power equilibrium point in the next cycle = the preset output power + the output power compensation value.

Step 1211: performing secondary processing on the ablation parameter.

The performing secondary processing on the ablation parameter in one embodiment is described as follows. The first power value calculation sub-module 312 calculates a power value needed to be outputted by the radio-frequency output apparatus 20 in the next cycle according to the current voltage, the current electric current, and the output power equilibrium point in the next cycle.

A specific calculation process includes:
calculating a second deviation, wherein the second deviation = the output power equilibrium point in the next cycle - the real-time power, for example, the second deviation = the output power equilibrium point in the next cycle - the real-time power;
calculating a first P deviation, wherein the first P deviation = the second deviation - a second deviation in a previous cycle;
calculating a first I deviation, wherein the first I deviation = the second deviation;
calculating a first D deviation, wherein the first D deviation = the second deviation - 2* the second deviation in the previous cycle + a second deviation in a further previous cycle which is a preceding cycle adjacent to and before the previous cycle;
calculating a power compensation value in the next cycle, wherein the power compensation value in the next cycle = P coefficient * the first P deviation + I coefficient * the first I deviation + D coefficient * the first D deviation; and
calculating the power value needed to be outputted in the next cycle, wherein the power value needed to be outputted in the next cycle = the real-time power + the power compensation value in the next cycle;
wherein the P coefficient, I coefficient, and D coefficient are values obtained from test data or empirical values.

Step 1212: acquiring an input control voltage.

Specifically, in one embodiment, since the correspondence relation between the input control voltage and the output power is nonlinear, the input control voltage corresponding to the output power can be obtained from a correspondence relation curve shown in Fig. 9 according to the output power. This further improves the output accuracy and stability in continuous output of the radio-frequency ablation instrument 100.

Step 1213: outputting energy.

Specifically, in one embodiment, the control apparatus 30 controls the radio-frequency output apparatus 20 to output the radio-frequency energy according to the input control voltage.

Step 1214: determining whether a condition of stopping the ablation is met.

Specifically, in one embodiment, the control apparatus 30 determines whether the stop button is triggered, whether the ablation duration is time out, whether the temperature is abnormal or whether the impedance is abnormal.

When the control apparatus 30 determines that one of the following events occur, the stop button is triggered, the ablation duration is time out, the temperature is abnormal and the impedance is abnormal, Step 1215 is performed. If not, Step 126 is repeated.

Step 1215: stopping the ablation.

Specifically, in one embodiment, the control apparatus 30 controls the radio-frequency output apparatus 20 to stop outputting the radio-frequency energy.

Step 1216: processing the ablation parameter.

In the constant-power ablation mode, specifically, in one embodiment, the step of processing the ablation parameter means that the real-time power calculation sub-module 313 calculates a real-time power of the target object according to the current voltage and the current electric current, wherein the real-time power is a product of the current voltage and the current electric current; and
the second output power value calculation sub-module 314 calculates the power value needed to be outputted by the radio-frequency output apparatus 20 in the next cycle with the PID algorithm according to the real-time power and the preset constant-ablation power point. A specific calculation process includes:
calculating a third deviation, wherein the third deviation = the preset constant-ablation power point - the real-time power;
calculating a second P deviation, wherein the second P deviation = the third deviation - a third deviation in a previous cycle;
calculating a second I deviation, wherein the second I deviation = the third deviation;
calculating a second D deviation, wherein the second D deviation = the third deviation -2* the third deviation in the previous cycle + a third deviation in a further previous cycle which is a preceding cycle adjacent to and before the previous cycle;
calculating a next output point compensation power value, wherein the next output point compensation power value =P coefficient * the second P deviation + I coefficient * the second I deviation + D coefficient * the second D deviation; and
calculating the power value needed to be outputted in the next cycle, wherein the power value needed to be outputted in the next cycle = the real-time power + the next output point compensation power value;
wherein the P coefficient, I coefficient, and D coefficient are values obtained from test data or empirical values.

Step 1217: acquiring an input control voltage.

An input control voltage is acquired.

Specifically, in one embodiment, since the correspondence relation between the input control voltage and the output power is nonlinear, the input control voltage corresponding to the output power can be obtained from a correspondence relation curve shown in Fig. 9 according to the output power. This further improves the output accuracy and stability in continuous output of the radio-frequency ablation instrument 100.

Step 1218: outputting energy.

Specifically, in one embodiment, the control apparatus 30 controls the radio-frequency output apparatus 20 to output the radio-frequency energy according to the input control voltage.

Step 1219: determining whether a condition of stopping the ablation is met.

Specifically, in one embodiment, the control apparatus 30 determines whether the stop button is triggered, whether the ablation duration is time out, whether the temperature is abnormal or whether the impedance is abnormal.

When the control apparatus 30 determines that one of the following events occur: the stop button is triggered, the ablation duration is time out, the temperature is abnormal and the impedance is abnormal, Step 1215 is performed. If not, Step 126 is repeated.

A specific process of implementing the above steps by the control apparatus 30 can be made reference to the relevant description in the embodiments shown in Figs. 1 to 12.

Therefore, in the present disclosure, the constant-temperature ablation mode or constant-power ablation mode can be selected according to requirements of the target object, so the present disclosure has a wider range of application. Moreover, the incremental algorithm, PID algorithm, and non-linear control are employed to control the real-time ablation energy output, which increases the real-time stability, and guarantees that the system is accurate and effective in both modes. Therefore, the real-time stability, safety, accuracy, stability, and practicality are greatly improved.

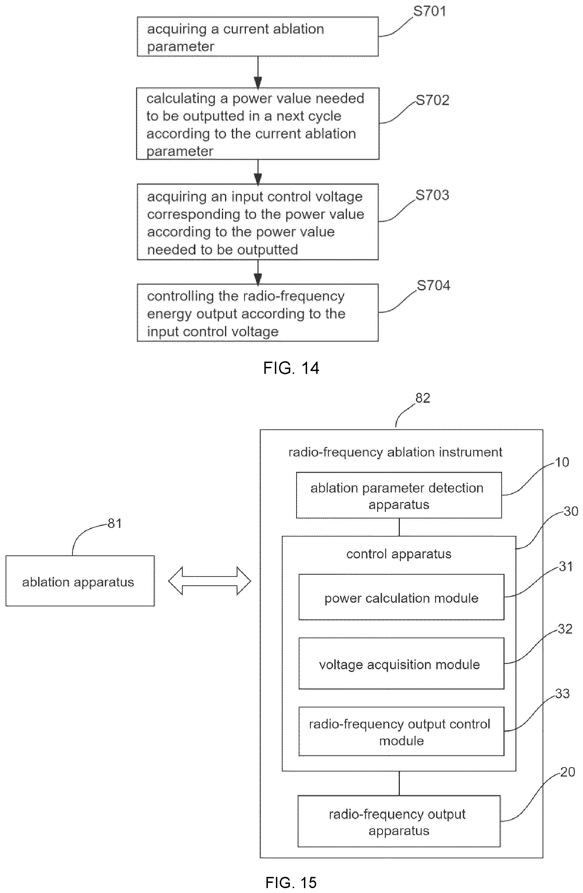

An embodiment of the present disclosure further provides a control method of a radio-frequency ablation instrument 100, which can be implemented by the control apparatus of the radio-frequency ablation instrument 100 described above. As shown in Fig. 14, the method mainly includes the following steps:
S701: acquiring a current ablation parameter;
S702: calculating a power value needed to be outputted in a next cycle according to the current ablation parameter;
S703: acquiring an input control voltage corresponding to the power value according to the power value needed to be outputted; and
S704: controlling the radio-frequency energy output according to the input control voltage.

The process of implementing Steps S701 to S704 can be specifically made reference to relevant descriptions of the radio-frequency ablation instrument 100 and the control method of the radio-frequency ablation instrument 100 in the foregoing embodiments shown in Figs. 1 to 13, and descriptions will not be repeated here again.

An embodiment of the present disclosure further provides a radio-frequency ablation system. As shown in Fig. 15, the system includes an ablation apparatus 81 and a radio-frequency ablation instrument 82. The ablation apparatus 81 is electrically connected to the radio-frequency ablation instrument 82, the radio-frequency ablation instrument 82 is configured to provide radio-frequency energy to the ablation apparatus 81, and the ablation apparatus 81 is configured to deliver the radio-frequency energy to a site to be ablated.

The specific structure and function of the radio-frequency ablation instrument 82 can be made reference to relevant descriptions of the radio-frequency ablation instrument 100 and the control method of the radio-frequency ablation instrument 100 in the foregoing embodiments shown in Figs. 1 to 14, and descriptions will not be repeated here again.

An embodiment of the present disclosure further provides a control apparatus of a radio-frequency ablation instrument. The specific structure and function of the control apparatus of a radio-frequency ablation instrument can be made reference to relevant descriptions of the control apparatus 30 in the embodiments shown in Figs. 1 to 12, and descriptions will not be repeated here again.

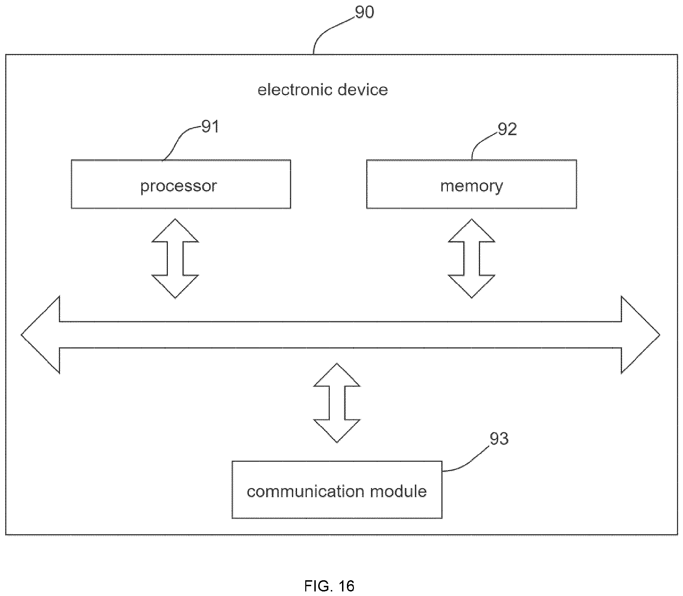

An embodiment of the present disclosure further provides an electronic device, which can be integrated in the radio-frequency ablation instrument 100 to serve as the control apparatus 30, or formed as a separate device from the radio-frequency ablation instrument 100 and configured to implement the function of the control apparatus 30 or the control method of the radio-frequency ablation instrument by data interaction with the radio-frequency ablation instrument 100. The electronic device may be any of various types of computer devices that are non-movable or movable or portable and capable of wireless or wired communication. As shown in Fig. 16, the electronic device 90 includes at least a processor 91, and a memory 92 on which program codes executable by the processor are stored. The processor 91 connected to the memory 92 invokes the executable program codes stored in the memory 92, to implement the control method of the radio-frequency ablation instrument 100 described in the embodiments shown in Figs. 12 to 14.

Further, the executable program codes can include computer instructions for implementing various function modules above (for example, the power calculation module 31, the voltage acquisition module 32, the radio-frequency outputted control module 33or the like).

As shown in Fig. 16, in another embodiment, the electronic device 90 may further include a communication module 93, which is configured to provide the electronic device 90 with an ability to communicate with an external device. The communication module 93 may include analog and digital input/output interface circuits, and a wireless communication circuit based on radio-frequency signals and/or optical signals. The wireless communication circuit in the communication module may include a radio-frequency transceiver circuit, a power amplifier circuit, a low-noise amplifier, a switch, a filter and an antenna. For example, the wireless communication circuit in the communication module may include a circuit for supporting near field communication (NFC) by transmitting and receiving near-field coupled electromagnetic signals. For example, the communication module may include a near-field communication antenna and a near-field communication transceiver. The communication module may further include a cellular phone transceiver and antenna, and a wireless LAN transceiver circuit and antenna, etc.

A specific process for implementing the control method of the radio-frequency ablation instrument 100 above by the electronic device 90 can be made reference to relevant descriptions in embodiments shown in Figs. 1 to 14, and descriptions will not be repeated here again.

An embodiment of the present disclosure further provides a computer storage medium, and specifically a computer readable storage medium, on which a computer program is stored. When the computer program is executed by the processor, the control method of the radio-frequency ablation instrument 100 as shown in Figs. 12 to 14 above is performed.

The computer readable storage medium can be configured in radio-frequency ablation instrument 100, and connected to the control apparatus 30 and the slave control apparatus 50. The computer program includes multiple computer instructions, which are implemented by the control apparatus 30, to realize the corresponding functions of the power calculation module 31, the voltage acquisition module 32, the radio-frequency outputted control module 33 and the like. Alternatively, the computer readable storage medium is configured in other computer devices than the radio-frequency ablation instrument 100, for example, the electronic device 90. Optionally, the computer readable storage medium is a non-temporary computer readable storage medium.

It should be noted that for ease of descriptions of the above method embodiments, they are all described as a series of actions. However, it is to be known by those skilled in the art that the present disclosure is not limited to the sequence of actions described, because some steps can be done in a different sequence or simultaneously according to the present disclosure. Further, it is to be known by those skilled in the art that embodiments described in the specification are all preferred embodiments, the actions and modules involved therein are not necessarily required for the present disclosure.

In the embodiments described above, emphasis has been placed on the descriptions of various embodiments. Parts of an embodiment that are not described in detail may be found in the descriptions of other embodiments.

In the embodiments provided in the present disclosure, it can be understood that the disclosed device may be implemented in other ways. For example, the device embodiments described above are merely illustrative. For example, the division of the units is merely a division of logical functions, and there may be additional divisions in actual implementation. For example, multiple elements or components may be combined or integrated into another system, or some features may be omitted, or not performed. Alternatively, the couplings or direct couplings or communicative connections shown or discussed with respect to one another may be indirect couplings or communicative connections via some interfaces, devices or units, which may be electrical, or otherwise.

The units described as separate components may or may not be physically separate, and the components shown as units may or may not be physical units, i.e. may be located in one place, or may be distributed over a plurality of network elements. Some or all of the units may be selected to achieve the objectives of the solution of the present embodiment according to practical requirements.

In addition, the functional units in the various embodiments of the present disclosure may be integrated into one processing unit, may be physically separate from each other or may be integrated in one unit by two or more units. The integrated units described above can be implemented either in the form of hardware, or software program modules.

The integrated unit, if implemented in the form of a software program module and sold or used as a stand-alone product, may be stored in a computer-readable storage medium. Based on such an understanding, the essence of the technical solution of present disclosure or part thereof contributing to the related art, or all or part of the technical solution can be implemented by a software product, The computer software product is stored in a memory, and includes a plurality of instructions executable by a computer device (for example, personnel computer, sever, or network device) to perform all or some of the steps in the methods according to various embodiments of the present disclosure. The memory includes: a USB flash disk, a read-only memory (ROM), a random access memory (RAM), a movable hard disk, a magnetic disk, an optical disc and various other media on which program codes can be stored.

It can be understood by those of ordinary skill in the art that all or some of the steps of the methods provided in the embodiments above may be performed by relevant hardware under instruction of a program that is stored in a computer readable memory, including a flash memory, a read only memory (ROM), a random access memory (RAM), a magnetic disk or an optical disc.

The above-described embodiments are merely illustrative of the technical solutions of the present disclosure, and are not intended to limit thereof. Although the present disclosure has been described in detail with reference to the foregoing embodiments, those skilled in the art will appreciate that the technical solutions of the above-mentioned embodiments can still be modified, or some of the technical features thereof can be equivalently substituted; and such modifications and substitutions do not cause the nature of the corresponding technical solution to depart from the scope of the embodiments of the present disclosure.

## Claims

1. A control method of a radio-frequency ablation instrument, comprising steps of:
acquiring a current ablation parameter;
calculating a power value needed to be outputted in a next cycle according to the current ablation parameter;
acquiring an input control voltage corresponding to the power value according to the power value needed to be outputted; and
controlling the radio-frequency energy output according to the input control voltage.

2. The method according to claim 1, further comprising a step of
selecting an ablation mode of the radio-frequency ablation instrument in response to an ablation mode selection signal, wherein the ablation mode comprises a constant-temperature ablation mode and a constant-power ablation mode, and the ablation mode selection signal is generated in response to a human-machine interaction operation.

3. The method according to claim 2, further comprising a step of
setting an ablation parameter according to an ablation parameter setting control signal, wherein the ablation parameter setting control signal is generated in response to a human-machine interaction operation, the parameter needed to be set in the constant-temperature ablation mode comprises at least a preset constant-temperature point and a preset output power, and the parameter needed to be set in the constant-power ablation mode comprises at least a preset constant-ablation power point.

4. The method according to claim 3, wherein the step of calculating a power value needed to be outputted in a next cycle according to the current ablation parameter comprises:
in the constant-temperature ablation mode, performing primary processing on the current ablation parameter;
performing secondary processing on the ablation parameter after the primary processing; and calculating a power value needed to be outputted in a next cycle according to the ablation parameter after the secondary processing.

5. The method according to claim 4, wherein the step of performing primary processing on the current ablation parameter comprises:
in the constant-temperature ablation mode, calculating an output power equilibrium point in a next cycle according to a current temperature of a target object and the preset constant-temperature point.

6. The method according to claim 5, wherein the step of calculating an output power equilibrium point in a next cycle according to a current temperature of the target object and the preset constant-temperature point comprises:
calculating the output power equilibrium point in the next cycle with an incremental algorithm according to the current temperature and the preset constant-temperature point.

7. The method according to claim 6, wherein the step of calculating the output power equilibrium point in the next cycle by an incremental algorithm according to the current temperature and the preset constant-temperature point comprises:
calculating a first deviation, wherein the first deviation = the preset constant-temperature point - the current temperature;
obtaining an output power compensation value by querying the incremental level according to the first deviation; and
calculating the output power equilibrium point in a next cycle according to the preset output power and the output power compensation value, wherein the output power equilibrium point in the next cycle = the preset output power + the output power compensation value.

8. The method according to any one of claims 4 to 7, wherein the step of performing secondary processing on the ablation parameter after the primary processing and calculating a power value needed to be outputted in a next cycle according to the ablation parameter after the secondary processing comprises:
calculating the power value needed to be outputted in the next cycle according to a current voltage at an output terminal of the radio-frequency output apparatus, a current electric current at the output terminal of the radio-frequency output apparatus and the output power equilibrium point in the next cycle.

9. The method according to claim 8, wherein the step of calculating the power value needed to be outputted in the next cycle according to a current voltage at an output terminal of the radio-frequency output apparatus, a current electric current at the output terminal of the radio-frequency output apparatus and the output power equilibrium point in the next cycle comprises:
calculating a real-time power according to the current voltage and the current electric current; and
calculating the power value needed to be outputted in the next cycle with a PID algorithm according to the real-time power and the output power equilibrium point in the next cycle.

10. The method according to claim 9, wherein the step of calculating the power value needed to be outputted in the next cycle with the PID algorithm according to the real-time power and the output power equilibrium point in the next cycle comprises:
calculating a second deviation, wherein the second deviation = the output power equilibrium point in the next cycle - the real-time power;
calculating a first P deviation, wherein the first P deviation = the second deviation - a second deviation in a previous cycle;
calculating a first I deviation, wherein the first I deviation = the second deviation;
calculating a first D deviation, wherein the first D deviation = the second deviation - 2* the second deviation in the previous cycle + a second deviation in a further previous cycle which is a preceding cycle adjacent to and before the previous cycle;
calculating a power compensation value in the next cycle, wherein the power compensation value in the next cycle = P coefficient * the first P deviation + I coefficient * the first I deviation + D coefficient * the first D deviation; and
calculating the power value needed to be outputted in the next cycle, wherein the power value needed to be outputted in the next cycle = the real-time power + the power compensation value in the next cycle;
wherein the P coefficient, I coefficient and D coefficient are values obtained from test data or empirical values.

11. The method according to any one of claims 4 to 10, wherein the step of calculating the power value needed to be outputted in the next cycle according to the current ablation parameter further comprises:
in the constant-power ablation mode, calculating the real-time power of the target object according to the current voltage at an output terminal of the radio-frequency output apparatus and the current electric current at the output terminal of the radio-frequency output apparatus; and
calculating the power value needed to be outputted in the next cycle with a PID algorithm according to the real-time power and the preset constant-ablation power point.

12. The method according to claim 11, wherein the step of calculating the power value needed to be outputted in the next cycle by the PID algorithm according to the real-time power and the preset constant-ablation power point comprises:
calculating a third deviation, wherein the third deviation = the preset constant-ablation power point - the real-time power;
calculating a second P deviation, wherein the second P deviation = the third deviation - a third deviation in a previous cycle;
calculating a second I deviation, wherein the second I deviation = the third deviation;
calculating a second D deviation, wherein the second D deviation = the third deviation -2* the third deviation in the previous cycle + a third deviation in a further previous cycle which is a preceding cycle adjacent to and before the previous cycle;
calculating a next output point compensation power value, wherein the next output point compensation power value =P coefficient * the second P deviation + I coefficient * the second I deviation + D coefficient * the second D deviation; and
calculating the power value needed to be outputted in the next cycle, wherein the power value needed to be outputted in the next cycle = the real-time power + the next output point compensation power value;
wherein the P coefficient, I coefficient and D coefficient are values obtained from test data or empirical values.

13. The method according to any one of claims 1 to 12, wherein a correspondence relation between the output power of the radio-frequency ablation instrument and the input control voltage is nonlinear, and the input control voltage is obtainable from a correspondence relation table of the output power vs the input control voltage according to the output power.

14. The method according to any one of claims 3 to 13, wherein
the parameter needed to be set in the constant-temperature ablation mode further comprises at least one of the following: an impedance range, an ablation voltage range, an ablation current range, and an ablation duration, the parameter needed to be set in the constant-power ablation mode further comprises at least one of the following: an impedance range, an ablation voltage range, an ablation current range, an ablation duration, and a temperature protection range; and the method further comprises:
controlling the radio-frequency output apparatus to stop the ablation or adjust the radio-frequency energy output when one of the following events is detected:
the current impedance in an ablation loop of the radio-frequency output apparatus goes beyond the impedance range;
the current voltage at an output terminal of the radio-frequency output apparatus goes beyond the ablation voltage range;
the current electric current at the output terminal of the radio-frequency output apparatus goes beyond the ablation current range;
the ablation duration goes beyond the set ablation duration; and
the current temperature of the target object goes beyond the temperature protection range.

15. The method according to any one of claims 1 to 14, further comprising:
starting the working of radio-frequency ablation instrument or stopping the working of the radio-frequency ablation instrument in response to an ablation start/stop control signal.

16. The method according to any one of claims 1 to 15, further comprising:
controlling to display an ablation interface, wherein the ablation interface is configured to display at least one of the set ablation parameter, the current ablation parameter and a waveform pattern of the current ablation parameter.

17. The method according to any one of claims 1 to 16, wherein the step of acquiring a current ablation parameter comprises:
acquiring the current ablation parameter in real time by a slave control apparatus of the radio-frequency ablation instrument.

18. A control apparatus of a radio-frequency ablation instrument, comprising:
a power calculation module, configured to acquire a current ablation parameter, and calculate a power value needed to be outputted by the radio-frequency ablation instrument in a next cycle according to the current ablation parameter;
a voltage acquisition module, configured to acquire an input control voltage corresponding to the power value according to the power value needed to be outputted; and
a radio-frequency output control module, configured to control the radio-frequency ablation instrument to output the radio-frequency energy according to the input control voltage.

19. The control apparatus according to claim 18, wherein the ablation mode of the radio-frequency ablation instrument comprises a constant-temperature ablation mode and a constant-power ablation mode, and the control apparatus selects one of the constant-temperature ablation mode and the constant-power ablation mode to perform the ablation in response to an ablation mode selection signal.

20. The control apparatus according to claim 19, wherein the control apparatus sets an ablation parameter according to an ablation parameter setting control signal, the parameter needed to be set in the constant-temperature ablation mode comprises at least a preset constant-temperature point and a preset output power, and the parameter needed to be set in the constant-power ablation mode comprises at least a preset constant-ablation power point.

21. The control apparatus according to claim 20, wherein in the constant-temperature ablation mode, the power calculation module comprises an output power equilibrium point calculation sub-module and a first output power value calculation sub-module, wherein the output power equilibrium point calculation sub-module is configured to calculate an output power equilibrium point of the radio-frequency ablation instrument in the next cycle according to a current temperature of a target object and the preset constant-temperature point; and the first output power value calculation sub-module is configured to calculate the power value needed to be outputted by the radio-frequency output apparatus in the next cycle according to a current voltage at an output terminal of the radio-frequency output apparatus, a current electric current at the output terminal of the radio-frequency output apparatus, and the output power equilibrium point in the next cycle.

22. The control apparatus according to claim 21, wherein the output power equilibrium point calculation sub-module is specifically configured to:
calculate the output power equilibrium point of the radio-frequency ablation instrument in the next cycle by an incremental algorithm according to the current temperature and the preset constant-temperature point.

23. The control apparatus according to claim 22, wherein the output power equilibrium point calculation sub-module is specifically configured to, calculate the output power equilibrium point of the radio-frequency ablation instrument in the next cycle with an incremental algorithm according to the current temperature and the preset constant-temperature point, comprising specifically:
calculating a first deviation, wherein the first deviation = the preset constant-temperature point - the current temperature;
obtaining an output power compensation value by querying the incremental level according to the first deviation; and
calculating the output power equilibrium point meeting constant temperature in the next cycle according to the preset output power and the output power compensation value, wherein the output power equilibrium point in the next cycle = the preset output power + the output power compensation value.

24. The control apparatus according to any one of claims 21 to 23, wherein the first output power value calculation sub-module is specifically configured to:
calculate a real-time power according to the collected current voltage and current electric current, and
calculate the power value needed to be outputted by the radio-frequency ablation instrument in the next cycle with a PID algorithm according to the real-time power and the output power equilibrium point in the next cycle.

25. The control apparatus according to claim 24, wherein the first output power value calculation sub-module is specifically configured to calculate the power value needed to be outputted by the radio-frequency output apparatus in the next cycle with the PID algorithm according to the real-time power and the output power equilibrium point in the next cycle, comprising specifically:
calculating a second deviation, wherein the second deviation = the output power equilibrium point in the next cycle - the real-time power;
calculating a first P deviation, wherein the first P deviation = the second deviation - a second deviation in a previous cycle;
calculating a first I deviation, wherein the first I deviation = the second deviation;
calculating a first D deviation, wherein the first D deviation = the second deviation - 2* the second deviation in the previous cycle + a second deviation in a period before the previous cycle;
calculating a power compensation value in the next cycle, wherein the power compensation value in the next cycle = P coefficient * the first P deviation + I coefficient * the first I deviation + D coefficient * the first D deviation; and
calculating the power value needed to be outputted in the next cycle, wherein the power value needed to be outputted in the next cycle = the real-time power + the power compensation value in the next cycle;
wherein the P coefficient, I coefficient, and D coefficient are values obtained from test data or empirical values.

26. The control apparatus according to any one of claims 21 to 25, wherein in the constant-power ablation mode, the power calculation module comprises a real-time power calculation sub-module and a second output power value calculation sub-module, wherein
the real-time power calculation sub-module is configured to calculate the real-time power of the target object according to the current voltage and the current electric current, and
the second output power value calculation sub-module is configured to calculate the power value needed to be outputted by the radio-frequency ablation instrument in the next cycle with the PID algorithm according to the real-time power and the present constant-ablation power point.

27. The control apparatus according to claim 26, wherein the second output power value calculation sub-module is specifically configured to:
calculate a third deviation, wherein the third deviation = the preset constant-ablation power point - the real-time power;
calculate a second P deviation, wherein the second P deviation = the third deviation - a third deviation in a previous cycle;
calculate a second I deviation, wherein the second I deviation = the third deviation;
calculate a second D deviation, wherein the second D deviation = the third deviation -2* the third deviation in the previous cycle + a third deviation in a further previous cycle which is a preceding cycle adjacent to and before the previous cycle;
calculate a next output point compensation power value, wherein the next output point compensation power value =P coefficient * the second P deviation + I coefficient * the second I deviation + D coefficient * the second D deviation; and
calculate the power value needed to be outputted in the next cycle, wherein the power value needed to be outputted in the next cycle = the real-time power + the next output point compensation power value;
wherein the P coefficient, I coefficient, and D coefficient are values obtained from test data or empirical values.

28. The control apparatus according to any one of claims 18 to 27, wherein a correspondence relation between the output power of the radio-frequency ablation instrument and the input control voltage is nonlinear, and the control apparatus obtains the input control voltage corresponding to the output power from a correspondence relation table of the output power vs the input control voltage according to the output power of the radio-frequency ablation instrument.

29. The control apparatus according to any one of claims 21 to 28, wherein the parameter needed to be set in the constant-temperature ablation mode further comprises at least one of the following: an impedance range, an ablation voltage range, an ablation current range, and an ablation duration; and wherein the parameter needed to be set in the constant-power ablation mode further comprises at least one of the following: an impedance range, an ablation voltage range, an ablation current range, and an ablation duration, and a temperature protection range; and
the control apparatus controls the radio-frequency output apparatus to stop the ablation or adjust the radio-frequency energy output when one of the following events occurs: the current impedance in the ablation loop of the radio-frequency output apparatus goes beyond the impedance range, the current voltage goes beyond the ablation voltage range, the current electric current goes beyond the ablation current range, the ablation duration goes beyond the set ablation duration, and the current temperature goes beyond the temperature protection range.

30. The control apparatus according to any one of claims 18 to 29, wherein the control apparatus starts the working of the radio-frequency ablation instrument or stop the working of the radio-frequency ablation instrument in response to an ablation start/stop control signal.

31. The control apparatus according to any one of claims 18 to 30, wherein the control apparatus controls a display screen to display an ablation interface, wherein the ablation interface is configured to display at least one of the a ablation parameter, the current ablation parameter, and a waveform pattern of the current ablation parameter.

32. The control apparatus according to any one of claims 18 to 31, wherein the control apparatus acquires the current ablation parameter from a slave control apparatus of the radio-frequency ablation instrument.

33. An electronic device, comprising a processor, and a memory configured to store instructions executable by the processor, wherein the processor is configured to perform the control method of a radio-frequency ablation instrument according to any one of claims 1 to 17 by implementing the executable instructions.

34. A radio-frequency ablation instrument, comprising an ablation parameter detection apparatus, configured to collect a current ablation parameter in real time; a radio-frequency output apparatus; and a control apparatus according to any one of claims 18 to 32, or an electronic device according to claim 33.

35. The radio-frequency ablation instrument according to claim 34, further comprising a human-machine interaction apparatus, wherein the human-machine interaction apparatus comprises an ablation mode selection module, configured to generate, in response to a human-machine interaction operation, a corresponding ablation mode selection signal.

36. The radio-frequency ablation instrument according to claim 34 or 35, wherein the human-machine interaction apparatus further comprises: an ablation parameter setting module, configured to generate, in response to a human-machine interaction operation, a corresponding ablation parameter setting control signal.

37. The radio-frequency ablation instrument according to any one of claims 34 to 36, wherein the ablation parameter detection apparatus comprises a temperature detection module, a voltage detection module and a current detection module respectively connected to the control apparatus, wherein the temperature detection module is configured to detect a current temperature of the target object; the voltage detection module is configured to detect a current voltage at the output terminal of the radio-frequency output apparatus; and the current detection module is configured to detect a current electric current at an output terminal of the radio-frequency output apparatus.

38. The radio-frequency ablation instrument according to any one of claims 34 to 37, wherein the ablation parameter detection apparatus further comprises an impedance detection module and ablation duration detection module respectively connected to the control apparatus, wherein the impedance detection module is configured to detect a current impedance in an ablation loop of the radio-frequency output apparatus, and the ablation duration detection module is configured to detect an ablation duration.

39. The radio-frequency ablation instrument according to any one of claims 35 to 38, wherein the human-machine interaction apparatus further comprises an ablation start/stop module, configured to generate, in response to a human-machine interaction operation, a corresponding ablation start/stop control signal.

40. The radio-frequency ablation instrument according to any one of claims 35 to 39, wherein the human-machine interaction apparatus further comprises a display screen.

41. The radio-frequency ablation instrument according to any one of claims 34 to 40, further comprising a slave control apparatus, configured to control the ablation parameter detection apparatus to acquire the current ablation parameter in real time.

42. A radio-frequency ablation system, comprising:
an ablation apparatus and a radio-frequency ablation instrument according to any one of claims 34 to 41,
wherein the ablation apparatus is electrically connected to the radio-frequency ablation instrument, the radio-frequency ablation instrument is configured to provide radio-frequency energy, and the ablation apparatus is configured to deliver the radio-frequency energy to a site to be ablated.

43. A computer readable storage medium, on which a computer program is stored, wherein when the computer program is executed by a processor, the control method of a radio-frequency ablation instrument according to any one of claims 1 to 17 is performed.
